# EUROPEAN PATENT SPECIFICATION

(11) **EP 4 170 669 B1**
(45) Date of publication and mention of the grant of the patent: **14.10.2026**
(21) Application number: 22202276.6
(22) Date of filing: 18.10.2022
(51) Int. Cl.: G16H 20/40

(54) **DEVICE AND METHOD FOR NEAR REAL-TIME PREDICTION OF A SCATTERED RADIATION MAP IN AN OPERATING ROOM**
VORRICHTUNG UND VERFAHREN ZUR FAST-ECHTZEIT-VORHERSAGE EINER STREUSTRAHLUNGSKARTE IN EINEM OPERATIONSSAAL
DISPOSITIF ET PROCÉDÉ DE PRÉDICTION EN TEMPS QUASI RÉEL D'UNE CARTE DE RAYONNEMENT DIFFUSÉ DANS UNE SALLE D'OPÉRATION

(30) Priority: 22.10.2021 EP 21306474
(43) Date of publication of application: 26.04.2023
(73) Proprietor: Centre National de la Recherche Scientifique, 75016 Paris (FR); Université de Strasbourg, 67000 Strasbourg (FR); INSTITUT NATIONAL DE LA SANTE ET DE LA RECHERCHE MEDICALE, 75654 Paris Cedex 13 (FR); Centre Hospitalier Régional et Universitaire de Brest, 29200 Brest (FR); Université de Brest, 29200 Brest (FR)
(72) Inventor: Bert, Julien, 29200 Brest (FR); Villa Arias, Matéo, 29200 Brest (FR); Padoy, Nicolas, 67000 Strasbourg (FR); Visvikis, Dimitris, 29200 Brest (FR)
(74) Representative: Novagraaf Technologies

(56) References cited:
- BOUZID DOUNIA ET AL: "Monte-Carlo dosimetry for intraoperative radiotherapy using a low energy x-ray source", ACTA ONCOLOGICA., vol. 54, no. 10, 24 March 2015 (2015-03-24), GB, pages 1788 - 1795, XP055908246, ISSN: 0284-186X, DOI: 10.3109/0284186X.2015.1016623
- TANABE HIROYOSHI ET AL: "Fast EUV lithography simulation using convolutional neural network", JOURNAL OF MICRO/NANOLITHOGRAPHY, MEMS, AND MOEMS, SPIE - INTERNATIONAL SOCIETY FOR OPTICAL ENGINEERING, US, vol. 20, no. 4, 1 October 2021 (2021-10-01), pages 41202, XP060149677, ISSN: 1932-5150, [retrieved on 20210924], DOI: 10.1117/1.JMM.20.4.041202
- AURALEE EDELEN ET AL: "Machine Learning for Orders of Magnitude Speedup in Multi-Objective Optimization of Particle Accelerator Systems", ARXIV.ORG, CORNELL UNIVERSITY LIBRARY, 201 OLIN LIBRARY CORNELL UNIVERSITY ITHACA, NY 14853, 19 March 2019 (2019-03-19), XP081581226
- PENG ZHAO ET AL: "A method of rapid quantification of patient-specific organ doses for CT using deep-learning-based multi-organ segmentation and GPU-accelerated Monte Carlo dose computing", vol. 47, no. 6, 3 April 2020 (2020-04-03), US, pages 2526 - 2536, XP055911075, ISSN: 0094-2405, Retrieved from the Internet <URL:https://onlinelibrary.wiley.com/doi/full-xml/10.1002/mp.14131> DOI: 10.1002/mp.14131
- SHAN HONGMING ET AL: "Synergizing medical imaging and radiotherapy with deep learning", vol. 1, no. 2, 23 June 2020 (2020-06-23), pages 021001, XP055881802, Retrieved from the Internet <URL:https://iopscience.iop.org/article/10.1088/2632-2153/ab869f> DOI: 10.1088/2632-2153/ab869f

## Description

### Technical Field

The invention relates to estimation of scattered radiation map in an operating room, especially in the context of interventional radiology.

It applies in particular to the prevention of risks linked to scattered radiation due to this type of radiology, by allowing a quasi-real time prediction of a scattered radiation map of the intervention or operating environment.

### Background

X-ray-guided procedures have gained popularity in the last decades. Modern fluoroscopic devices allow real-time guidance of a variety of minimally invasive procedures. However, their complexity implies the acquisition of extensive volumes of images over extended fluoroscopic times. During this process, medical personnel need to stand near the x-ray device and the patient, exposing themselves to scatted ionizing radiation.

Radiations may be further used in the context of interventional radiology. Interventional radiology refers to all medical procedures performed by radiologists under radiological control, allowing the treatment or invasive diagnosis of numerous pathologies. The principle of interventional radiology is therefore to access a lesion located inside the body in order to carry out a diagnostic (sampling, for example) or therapeutic act (aimed at treating, repairing, closing, etc.).

Thus, in the context of therapeutic intervention, interventional radiology represents in certain cases an alternative to conventional surgical treatment, by making it possible to intervene inside the body, via the natural channels and without making surgical openings

Different technologies can be used such as, for example, fluoroscopy, X-ray scanning, ultrasound scanning, MRI (magnetic resonance imaging), etc.

Interventional radiology therefore involves irradiating portions of the human body from within the body and for the duration of the procedure. Some radiations may be scattered out of the body of the patient, due to interaction of x-ray radiations with human tissues.

Given the frequency of the interventions, staff reaches significant long-term levels of cumulative radiation. As a result, they may undergo some biological side-effects such as radiation-induced cancers, skin lesions, and eye lens damages.

Some recommendations have been made to employ specific protective equipment, such as lead aprons, thyroid shields, goggles, and gloves to reduce the amount of absorbed radiation in the body. However, studies have revealed improper and incomplete use of these accessories.

For controlling cumulative radiation dose levels, staff must systematically wear passive thermoluminiscent dosimeters, TLD, badges. Such badges are placed at the chest level below the lead apron. The absorbed dose is analyzed after the procedure. Yet, research has shown that the dose in various parts of the body may vary significantly for the same kind of intervention. Wearing multiple TLDs at various body parts would turn impractical from an ergonomic point of view.

Therefore, there is no solution in the state of the art that is both near-real time and sufficiently accurate.

Document BOUZID DOUNIA ET AL: "Monte-Carlo dosimetry for intraoperative radiotherapy using a low energy x-ray source", ACTA ONCOLOGICA., vol. 54, no. 10, 24 March 2015 (2015-03-24), pages 1788-1795, XP055908246,GB ISSN: 0284-186X discloses a Monte-Carlo simulation platform that computes three-dimensional voxel-based dose distributions for an intraoperative x-ray radiotherapy system, including modelling of the x-ray source, applicators and patient-specific tissue heterogeneities to obtain patient-specific 3D dose distributions for IORT.

### Summary of the invention

An objective of the present invention is to provide a method and a system that at least partially alleviates the above-mentioned drawbacks.

More particularly, according to embodiments, it aims to provide a scattered radiation map in real time, or quasi-real time, i.e. in a time allowing the practitioner to control his/her irradiation during the operation. S/he can thus react during the operation according to the dynamically provided information.

A first example embodiment concerns a method for training a multilayer neural network in view of obtaining a predicted scattered radiation map of an operating environment, said method comprising submitting to said multilayer neural network a learning set comprising associations between a first input tensor comprising data of a first radiology image of a patient's intervention area, and first acquisition parameters of said interventional radiology device, and labels corresponding to an scattered radiation map obtained by simulation by a simulation module from the said first radiology image and the said first acquisition parameters, and iteratively training said multilayer neural network by minimizing a cost function

In preferred embodiments, the invention comprises one or more of the following features which may be used separately or in partial combination with each other or in total combination with each other:
- during the learning phase, a plurality of data of the learning set is generated for a first radiology image by varying said first acquisition parameters among possible parameters;
- said simulation is performed by a Monte-Carlo method adapted for a graphics processor;
- said neural network is of U-Net type;
- said neural network consists of a first sub-network having as input the data of said first or second radiology image, and comprising a first succession of convolution and pooling layers and a second succession of deconvolution and convolution layers, such that the output of said neural network is of the same size as said input, and a second sub-network having as input said first or second acquisition parameters, respectively, and whose output is concatenated with the output of the last pooling layer of said first succession;
- said input tensor is a concatenation of said data of a first radiology image and said first acquisition parameters;
- said radiology image is a computerized tomography scan;
other embodiments of the invention relates to a method for generating a predictive scattered radiation map of an operating environment, comprising a step of training a multilayer neural network as previously defined, and a step of predicting said predictive scattered radiation map by submitting to said multilayer neural network a second input tensor and retrieving said predictive scattered radiation map prediction, said second tensor comprising data of a second radiology image of said given patient and of second acquisition parameters acquired from an radiology device.

According to other embodiments, the invention relates to a computer readable medium encoding a machine-executable program of instructions to perform a method as previously described.

According to other embodiments, the invention relates to a system for training a multilayer neural network in view of obtaining a predicted scattered radiation map of an operating environment, said system being adapted for submitting to said multilayer neural network a learning set comprising associations between a first input tensor comprising data of a first radiology image of a patient's intervention area, and first acquisition parameters of said interventional radiology device, and labels corresponding to an scattered radiation map obtained by simulation by a simulation module from the said first radiology image and the said first acquisition parameters, and for iteratively training said multilayer neural network by minimizing a cost function.

Further features and advantages of the invention will become apparent from the following description of a preferred embodiment of the invention, given by way of example and with reference to the attached drawings.

### Brief Description of the Figures

Some embodiments are now described, by way of example only, and with reference to the accompanying drawings, in which:
- figure 1 schematically represents an example of functional architecture for a learning phase according to an embodiment of the invention.
- figure 2 schematically illustrates an example of functional architecture for a prediction phase according to an embodiment of the invention
- figure 3 schematically illustrates a basic architecture of a classical neural network.
- figure 4 schematically illustrates an example of a multilayer neural network according to an embodiment of the invention.

### Description of Embodiments

One of the aims of the invention lies in obtaining a scattered radiation map of an operating environment during radiology of a given patient.

The operating environment may be the operating room, OR, or operating theater, or a part of it surrounding the operation and where the risks are high in view of radiation level.

This scattered radiation map associates a quantity, or dose, of radiation with points in space contained in an environment surrounding the patient and the medical staff impacted by the radiology. These points are more or less numerous according to a chosen sampling of the area.

The scattered radiation map is based on data acquired during the operation involving interventional radiology.

According to the invention, the scattered radiation map is obtained quickly enough to allow its production to the practitioner during the operation while remaining relevant to the data acquired. In other words, the radiation taking place between the time of data acquisition and the production of the scattered radiation map can be considered as negligible.

The production of the scattered radiation map may allow the practitioner to react to the information represented by this map during the operation. This can be referred to as real-time, in the sense that a human practitioner is able to react dynamically to the scattered radiation map calculated from measured data.

According to the invention, the generation of a scattered radiation map is based on a predictive model constituted by a multilayer neural network.

From a very high-level point of view, multi-layer neural networks can be seen as black boxes whose internal parameters must be adjusted during a learning (or training) phase by presenting them with both input data and a desired output (or "label"). The error between this desired output and the 'natural' output of the network enables the parameters to be adjusted slightly in order to reduce the error. By presenting a large number of such "input data / desired output" pairs, the network learns to react correctly and provide a good output when presented with new, unlabeled input data.

According to an embodiment of the invention, the neural network used may be a multilayer perceptron. Other neural network architectures may be possible. In particular, a convolutional neural network can be used (ConvNet or CNN).

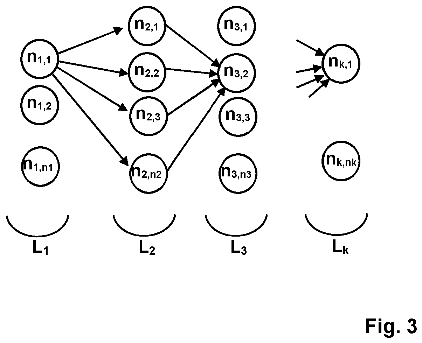

An example of a multilayer perceptron is shown in figure 3.

The multilayer perceptron (MLP) is a type of artificial neural network organized in several layers in which information flows from the input layer L₁ to the output layer Lₖ only; it is thus a feedforward network. Each layer L₁, L₂, L₃...Lₖ consists of a variable number of neurons, respectively n₁, n₂, n₃...nₖ. The neurons of the last layer (called "output") are the outputs of the neural network and represent a prediction of the model in response to an input provided on the L₁ layer.

In a multi-layer perceptron, each neuron n_{i,j} is connected as an output to all the neurons of the next layer Lᵢ₊₁. Conversely, it receives as input the outputs of all the neurons in the previous layer Lᵢ₋₁. In Figure 3, for clarity, only some connections are represented by oriented arrows.

Each connection is associated with a weight. The set of weights form the internal parameters of the neural network. They must be determined during a learning (or training) phase and then allow the prediction of output values, by generalization, from a new input vector presented on the L₁ input layer.

Each neuron n_{i,j} classically performs a weighted sum of these inputs by the weights of the associated connections and then applies an activation function to this sum.

Several techniques exist to determine the internal parameters of the network, the thresholds, by learning. One example is the Stochastic Gradient Descent (SGD) algorithm, described for example in Le Cun, Yann A., et al. "Efficient backprop. Neural networks: Tricks of the trade", Springer Berlin Heidelberg, 2012. 9-48. Another example is ADAM, originally described in Diederik P. Kingma and Jimmy Lei Ba. "Adam: A method for stochastic optimization". 2014. arXiv:1412.6980v9, or RMSprop, described in particular in Tijmen Tieleman and Geoffrey Hinton, "Lecture 6.5-rmsprop: Divide the gradient by a running average of its recent magnitude", COURSERA: neural networks for machine learning, 4(2):26-31, 2012.

Also, the process of obtaining a scattered radiation map includes a learning phase allowing to determine the internal parameters of the multilayer neural network.

Once these have been determined, the multilayer neural network constitutes a predictive model that can be used in a prediction phase during an intervention (or operation).

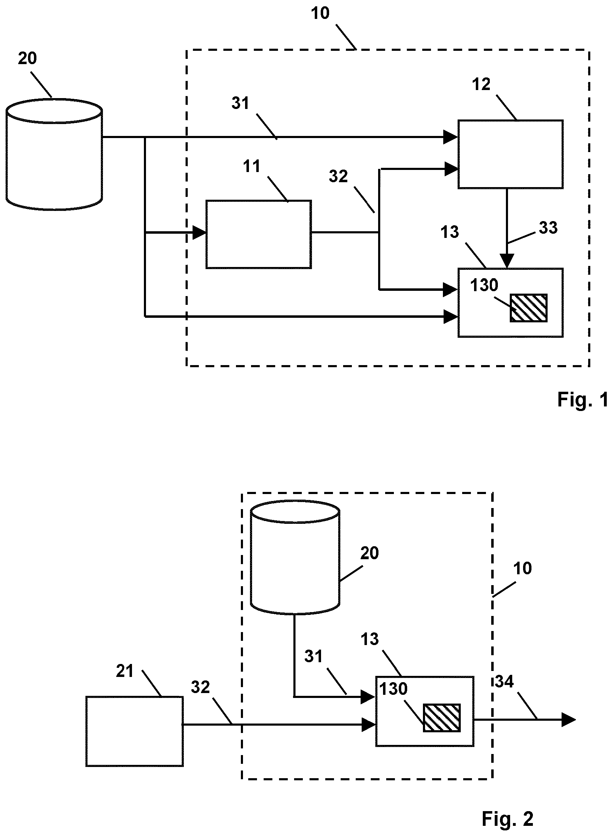

Figure 1 illustrates such a learning phase according to an embodiment of the invention.

According to the invention, the learning phase consists of submitting the multilayer neural network 13 to a learning set comprising associations between a learning input tensor itself comprising data from a radiology image 31 of a patient's intervention area, and acquisition parameters 32 of the interventional radiology device, and labels corresponding to a scattered radiation map 33 obtained by simulation, by a simulation module 12, on the basis of the radiology image 31 and of these acquisition parameters 32. The learning set may be stored in a database or data repository 20.

In a conventional manner, this learning phase is carried out before and outside the operation involving radiology.

The learning phase is based on a set of radiology images.

According to embodiments, the radiology images are three-dimensional images.

However, according to some other embodiments, other options may be contemplated. For instance, 2D images may be considered, the volume of the patient's intervention area captured by the radiology device being represented by a set of 2D images, each of them representing a layer of the volume.

Also, 4D images may be considered, the fourth dimension being the time (video stream).

Experiments have shown that it is fasted to predict a 3D image once by a multilayer neural network than to predict sequentially a succession of 2D images composing a 3D image.

In the following, the wording "three dimensional images" will be preferred, but the scope of the patent encompasses 2D and 4D images as well.

The radiology images (e.g. three-dimensional images) captures an intervention area of the patient related to the radiology (e.g. interventional radiology), i.e. locations of the patient's body where the radiology is aimed to produce effects.

These radiology images (e.g. three-dimensional images) may be of different kinds. For example, it may be a CT scan or computerized tomography (CT). This is a medical imaging technique which consists of measuring the absorption of X-rays by tissues and then, by computer processing, digitizing and finally reconstructing 2D or 3D images of anatomical structures. To acquire the data, the tomographic or "slice" analysis technique is used, by subjecting the patient to the scanning of an X-ray beam.

Typically, such a 3D image is taken prior to any intervention (or operation) in order to plan it. It is therefore possible to easily acquire a large number of such 3D images to form the training set.

Moreover, for each of these 3D images, acquisition parameters 32 of the interventional radiology device to be used for the operation can be associated. The radiology device (e.g. interventional radiology device) is designed to acquire an image, generally two-dimensional, of a patient's intervention area, that is a function of these acquisition parameters.

In practice, the interventional radiology device generates a two-dimensional image stream for the practitioner. It should be noted that these images are distinct from the one, 31, acquired before the intervention.

The acquisition parameters are primarily the three-axis coordinates, x,y,z, of the interventional radiology device, the angles α, β of orientation of the device, and the voltage, KV, of the device's tube. α represents, rotation about the longitudinal axis of the patient while β represents rotation about the horizontal axis.

Depending on the type of radiology device, other parameters may be used.

According to the invention, an input tensor of the neural network 13 may be formed by concatenating data from a CT scan image and acquisition parameters (x, y, z, α, β, KV), 32.

According to an embodiment of the invention, for each three-dimensional image 31, a plurality of training set data is generated by varying the acquisition parameters (x, y, z, α, β, KV) among possible parameters.

To do this, excursion intervals can be defined for each of the parameters x, y, z, α, β, KV as well as respective sampling rates. Thus, a large number of possible parameterizations can be defined for the same image for which a scattered radiation map 33 can be simulated.

Each three-dimensional image 31/acquisition parameters 32 pair can be produced at the input of a simulator 12 which provides a simulated scattered radiation map 33 at the output. This scattered radiation map 33 may be submitted as a label to the neural network 13.

In other words, at each iteration of the learning phase, the neural network 12, is provided with:
- an input tensor comprising a three-dimensional image 31 and acquisition parameters 32;
- associated with a label consisting of a scattered radiation map 33 simulated on the basis of this same three-dimensional image 31 and these same acquisition parameters 32.

In other words, the neural network allows correlation between the three-dimensional image data and the acquisition parameters (x, y, z, α, β, KV), 32 with corresponding acquisition maps.

According to the invention, the input tensor of the neural network contains only data produced by the interventional radiology device. No complex pre-processing needs to be performed in order to transform this acquired data into other data (apart from possible simple pre-processing such as re-scaling, re-sampling, etc. which do not change the nature of the data).

As a result, the training set is inexpensive to build, and a large number of labelled examples can be produced by simply retrieving three dimensional images. This training set can be further enriched by varying the acquisition parameters according to an embodiment of the invention.

It is neither necessary to deploy costly pre-processing that would slow down the constitution of a sufficiently large learning base, nor to obtain voxelized 3D models of each patient organ. The latter requirement is indeed detrimental to the constitution of a large learning base.

However, it is important that the training base is large enough to allow the neural network to converge to a stable state that minimizes its cost function, so that it can train an effective predictive model. A training base that is too small will either give erroneous or very inaccurate predictions, or correct predictions but on examples that differ only slightly from those in the training set (reduced generalization capacity).

Thus, even with the invention, it is possible to take advantage of available databases collecting patient data in order to perform this learning phase.

For example, a database of CT scan images of the pancreas exists and is documented in the article H. R. Roth, L. Lu, A. Farag, H.-C. Shin, J. Liu, E. Turkbey, R. M. Summers, "Data from Pancreas-CT", 2016. This database contains 82 images of 512 x 512 pixels, spaced in depth between 0.7 and 1 mm. From this database, three-dimensional images with a voxel resolution of 4 min x 4 min x 2 min can be redefined.

A simulation module 12 may perform a simulation of the behavior of the particles emitted by the interventional radiology device according to its acquisition parameters 32 in the patient's intervention area as defined by the three-dimensional image 31.

This simulation may be a simulation according to a Monte-Carlo method.

In particular, it may be a simulation according to a Monte Carlo method based on a GPU. For example, the GGEMS platform, for "GPU GEant4-based Monte Carlo Simulation", as described in the article by J. Bert, H. Perez-Ponce, Z. El Bitar, S. Jan, Y. Boursier, et al, "Geant4-based Monte Carlo simulations on GPU for medical applications" in Physics in Medicine and Biology, IOP Publishing, 2013, 58, pp.5593-5611, can be used. The objective of GGEMS is to provide a flexible platform for the simulation of various medical applications. The simulation algorithm can also be modified to better fit a platform running on a GPU.

In a concrete case of experimentation of the method of the invention, the source constituted by the interventional radiology device can be defined as forming a conical radiation of KV value. For each simulation, the behavior of 10⁷ particles can be simulated in a time of about 6 seconds on a GPU of the NVDIA 1080 GTX Ti type.

According to an embodiment of the invention, the acquisition parameters can be varied for each image in order to increase the population of the resulting training set.

Typically, the angles α, β vary in the ranges [-50°, 90°] and [-30°, +30°] respectively with steps of 2°.

The position of the interventional radiology device can vary in the intervals [-30cm; +30cm] for the three parameters x, y, z, with steps of 5 cm.

Finally, the tube voltage KV can be varied in the range [80kV, 120kV] with a step of 10kV.

These values of intervals and steps have been determined by studying the technical data of interventional radiology devices and by feedback from practitioners (surgeons, radiologists, etc.).

The simulation module 12 provides a scattered radiation map 33. This radiation map is a three-dimensional image that associates points in space within a simulated environment with radiation doses.

The simulated scattered radiation maps 33 form labels for the neural network 130 implemented by a neural network module 13.

In a manner known per se, training involves calculating an output of the neural network 130 as a function of the input tensor to measure a cost function (or loss function) that estimates a distance between that output and the associated label, or simulated scattered radiation map, that is desired output. The value of this cost function shall be minimized at each step of the iterative training process. In other words, the difference between these two values (in this case two tensors), evaluated by the cost function, is used to modify the parameters of the neural network in general by a gradient descent method as previously explained.

According to an embodiment of the invention, the cost function may be the evaluation of the mean square error between the output tensor of the neural network 13 and the desired output represented by the simulated scattered radiation map (i.e. labels).

The comparison between the predicted scattered radiation map, E_{pred} and the simulated scattered radiation map, E_{MC} can be done voxel by voxel.

So, a cost function based on mean squared error, MSE, can be written as: $L \left({E}_{pred}, {E}_{MC}\right) = \frac{1}{M} \frac{1}{N} {\sum }_{i}^{M} {\sum }_{j}^{N} {\left({E}_{pred}-{E}_{MC}\right)}^{2}$ wherein N represents the total number of voxels in a scattered radiation map (both simulated and predicted ones having the same size), and M represents the number of radiation maps in the training set (or a subset of the latter).

According to an embodiment, logarithmic scaling is applied to both the predicted scattered radiation map, E_{pred} and the simulated scattered radiation map, E_{MC}.

For instance, the scaled maps can be written: ${E}_{MC}^{′} = 100 \times {log}_{10} \left({E}_{MC}\right)$ ${E}_{pred}^{′} = 100 \times {log}_{10} \left({E}_{pred}\right)$

This enables reducing the local variability of the scattered radiation maps, which makes the loss function more suitable to be optimized. The optimization is then performed to learn a transformed radiation map ${E}_{pred}^{′}$ with the following loss function: $L \left({E}_{pred}^{′}, {E}_{MC}^{′}\right) = \frac{1}{M} \frac{1}{N} {\sum }_{i}^{M} {\sum }_{j}^{N} {\left({E}_{pred}^{′}-{E}_{MC}^{′}\right)}^{2}$

In the evaluation stage, the real values of the radiation maps can be retrieved by applying the inverse function: ${E}_{pred} = {10}^{\frac{{E}_{pred}^{′}}{100}}$

The learning mechanism can be optimized for example by the ADAM algorithm, described in D. P. Kingma and J. Ba, "Adam: A Method for Stochastic Optimization", CoRR, abs/1412.6980 (2014).

The neural network 13 may be implemented on a TensorFlow platform using the Keras API.

According to an embodiment of the invention, the neural network is a convolutional self-encoding multilayer neural network.

More particularly, the neural network may be a modified version of the u-net type neural network.

The u-net neural network is a convolutional network, first described in Olaf Ronneberger, Philipp Fischer, Thomas Brox, "U-net: Convolutional networks for biomedical image segmentation", in International Conference on Medical Image Computing and Computer-Assisted Intervention, pages 234-241, Springer, 2015.

A 3D version was then proposed in O. Cicek, A. Abdulkadir, S. S. Lienkamp, T. Brox, and O. Ronneberger, "3D U-net: learning dense volumetric segmentation from sparse annotation" in Lect. Ronneberger, "3D U-net: learning dense volumetric segmentation from sparse annotation" in Lect. Notes Computing Science, vol. 9901 LNCS, pages 424-432, 2016.

U-net network is further explained in the dedicated Wikipedia page: https://en.wikipedia.org/wiki/U-Net

According to an embodiment of the invention, the neural network is based on the 3D U-net architecture, but with the major a modification consisting in removing the "copy and crop" and "concat" mechanisms. The underlying rationales for that is that, unlike other U-net applications, in the present situation the input and the output are of different natures: the input comprises images of the patient's environment, whereas the output is a scattered radiation map, thus related to the operating environment. The scale of the 3D space represented by these two types of images are therefore different and copying/cropping data from the input towards to output along the U-net architecture would be meaningless and would jeopardize the efficiency of the network to learn and to predict good results.

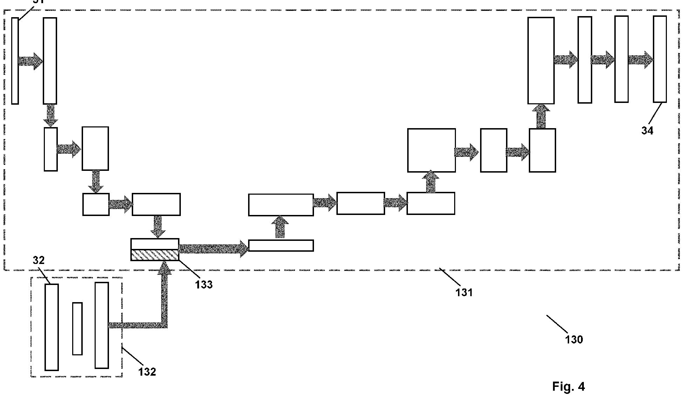

Figure 4 illustrates an example of such an embodiment.

In particular, the neural network 130 of the neural network module 13 is composed of a first subnet 131 corresponding to a 3D u-net and a second subnet 132 whose output is concatenated within the first subnet.

The first sub-network 131 itself comprises a first succession of layers constituting an encoding path (or contraction, or subsampling) and a second succession of layers constituting a decoding path (or expansion, or oversampling).

The encoding path is a succession of convolution layers and pooling layers.

More specifically, the encoding path may comprise successions of two successive 3x3x3 convolution layers, each followed by a ReLU activation and a 2x2x2 maxPooling layer. At each subsampling (or encoding) stage, the number of filters (or features) is doubled, starting with 8 for the first convolution.

The first layer of this encoding path takes as input the tensor formed by the three-dimensional image data 31.

The pooling operation reduces the dimensionality and captures the most relevant filters or features.

In Figure 4, the horizontal arrows to the right correspond to these convolution operations and the downward arrows correspond to maxPooling operations.

Similarly, the decoding path (right in Figure 4) has a succession of deconvolution and convolution layers.

The deconvolutions (or transposed convolutions, or oversampling), for example of 2x2x2 are represented by the upward arrows, while the convolutions, for example of 3x3x3 are represented by the rightward arrows in the same way as for the decoding channel.

In this way, an output (i.e. the scattered radiation map 34) of the same size as the input 31 can be obtained. However, as stated above, the semantics of the input and output images are different.

As illustrated in the figure 4, the pluralities of pooling (forming a subsampling) and then oversampling operations give the network a U-shaped (functional) form which gives it its usual name of "U-net".

The last layers of the encoding path and the first layers of the decoding path can also be considered as forming a bottleneck.

According to this embodiment, the neural network comprises, in an original way, a second subnetwork 132 which is coupled with the first subnetwork 131 at the level of this bottleneck.

This second subnet 132 takes as input the acquisition parameters 32 of the radiology device (e.g. the interventional radiology device).

This second sub-network may architecturally be a network of a few fully connected layers (here 3 in Figure 4). The output tensor 133 is concatenated with the output of the last pooling layer of the encoding path. This tensor has the same number of units (12x12x12=1728) as the output tensor of this last pooling layer in order to allow their concatenation.

According to an embodiment, the number of filters (or "features") for the convolution and deconvolution operations have been empirically reduced by a factor of 8 compared to the original 3D u-net, in order to allow faster predictions (in the prediction phase) compatible with a real-time requirement. The idea of doubling the number of filters before pooling operations has however been retained.

According to one embodiment, another distinctive feature compared to the u-net 3D scheme is the use of a padding mechanism to obtain an output of the same size as the input.

Padding aims in avoiding the edge effect of the recurrent convolution processes applied to the images.

More precisely, pixels around the image edge cannot be process due to the convolution.

For example, in case of a 3x3x3 convolutional filter, the edge around the image after processing is cropped with a size of 1 pixel. Consequently, the size of the image is reduced by two pixels on each dimension after every convolution, and it is not possible to recover this dimension on the decoder path.

This may be avoided by applying zero-padding to the input image before each convolution operation. The zero-padding consists of adding pixels with zeros value around the edge of the image.

In order to optimize the learning time, different mechanisms can be implemented.

For example, the training can be divided into two sub-phases.

In a first sub-phase, only a subset of the plurality of training set data obtained by varying the acquisition parameters among possible parameters is used. This subset is substantially smaller than this plurality.

For example, the module 11 generates in this sub-phase only a smaller number of parameters but preferably evenly distributed, for example using a larger step size.

For example, the orientation angles vary in 10° steps; the x,y,z position of the source varies in 15 cm steps and the tube voltage KV varies in 40kV steps.

This first sub-phase allows the state of the neural network to quickly converge to an acceptable state.

In a second sub-phase, the entire training set is used (i.e. with smaller steps, as previously indicated).

The training set is specific to a particular area of intervention: for example, specific training sets may be formed for the head and neck, for the thorax, for the pelvis, etc. A training set may be formed for the head and neck, for the thorax, for the pelvis, etc. A training set can also be formed for the whole body.

Each training set leads to a specific state of the neural network 13. This state is materialized by the values of all its parameters (synaptic weights, etc.) and thus constitutes a specific predictive model.

Once constituted, these predictive models (state of the neural network 13) is stored for use in the prediction phase.

During a prediction phase, a first step may therefore be to choose the appropriate predictive model from a library of predictive models. This step can be manual: the practitioner chooses the intervention area, which leads to the selection of the associated predictive model. According to other embodiments, this step may be automatic by applying image recognition algorithms allowing automatic recognition of an area of intervention.

This prediction phase consists in applying the predictive model for a given patient in order to predict a real-time scattered radiation map to help or alert the practitioner during radiology.

This phase is illustrated in Figure 2.

During the intervention, the radiology device 21 generates an acquisition parameter stream 32. This stream is determined, for example, by a sampling rate at which it transmits the acquisition parameters to the radiation map generation system, 10.

The parameters may be the 6-tuple previously described (x,y,z,α,β,KV), or any set of parameters specific to the type of radiology device. In any case, these parameters must be the same as those used for the learning phase.

These parameters evolve over time during the intervention, depending on the actions of the practitioner. Their values influence the radiation scattered from the patient towards the operation environment, in particular the area of the patient for the parameters (x,y,z,α,β) and the instantaneous dose for the parameter KV.

According to other embodiments, these parameters may be different ones, and of a different nature as well. For instance, in case of nuclear imagery, these parameters may include rather a dose (in Bq/cc) of nuclear elements injected into the patient and the time elapsed between the injection and the current time.

Furthermore, the system 10 is provided to prepare an input tensor comprising data of a radiology image 31 of the given patient, e.g. a three-dimensional. This radiology image is of the same nature as those used in the learning phase. It may be a pre-operative image of the patient, stored in the data repository 20, or an image acquired during the radiology operation. According to the invention, this image can be unique during the operation (only the parameters change over time).

The input tensor also includes parameters for acquiring the flow generated by the interventional radiology device 21.

Sampling may be applied to take only a sampled portion of the flow to construct this input tensor. The sampling may be determined based on the computation time for each prediction.

Once this input tensor has been prepared and submitted as an input to the neural network 13, a prediction of the scattered radiation map 34 is beretrieved as an output from the neural network 13.

This scattered radiation map 34 is of the same type as those provided as labels during the learning phase. In particular, it provides an estimated radiation dose per unit volume of the operation environment during the radiology operation.

This scattered radiation map can be seen as a 3D image in which each voxel is associated with a radiation dose (instead of a colorimetric data as in a classical 3D image).

Experimental measurements carried out by the inventors have made it possible to estimate that around 50 scattered radiation map per seconds can be generated with a standard GPU graphics card, meaning scattered radiation maps can be generated in around 20 ms.

This scattered radiation map can be used to assist the practitioner(s) during his intervention on the patient.

In particular, it can be displayed on a screen so that the practitioner can view it during the procedure. Radiation doses can be represented by associating different colors for intervals of dose values.

Since the time to calculate a new map is extremely short, the display can be refreshed (about 50 maps per second), so that the map displayed corresponds to the present time for the practitioner, allowing him/her to monitor the map in real time.

Also, alerts (visual, acoustic...) can be triggered if radiation doses exceed certain predetermined thresholds for certain sub-areas of the operation environment.

For example, it may be possible to visually highlight areas that may be problematic, either because the associated radiation dose has exceeded a threshold or because it shows too much radiation dose growth, suggesting that the threshold may be exceeded in the future.

Furthermore, the error inherent in a neural network prediction is very reasonable and less than 5% compared to a reference Monte Carlo simulation.

Moreover, as mentioned previously, the method uses only medical imaging data that is easy to obtain in the training phase, so that training sets can be constituted for various parts of the body that are sufficiently large to allow the neural network to converge well and thus obtain good qualitative performance in the prediction phase.

In one embodiment, the system 10 may be implemented by a set of circuits co-located in a centralized server or distributed within a distributed server or among a set of servers. This set of servers may comprise "server farm" or "cloud computing" arrangements. This server may also be implemented on a single computer located in the operating room.

This system 10 may have means of communication with remote servers in order to retrieve three-dimensional images of patients, both in the learning phase and in the prediction phase (in order to obtain a pre-operative image of the patient on whom the operation is to be performed).

The system may also have means of communication with the interventional radiology device 21 in order, in particular, to retrieve the acquisition parameters.

These communication means may be in accordance with the technologies of the state of the art. In particular, they may be wired (Ethernet, USB, etc.) or radio (Wi-Fi, Bluetooth, 3G/4G/5G cellular, etc.) communication means.

Of course, the present invention is not limited to the examples and the embodiments described but is defined by the claims. In particular, it is susceptible to numerous variants accessible to the person skilled in the art.

## Claims

1. Method for training a multilayer neural network (130) in view of obtaining a predicted scattered radiation map of an operating environment during interventional radiology of an intervention area of a given patient using an interventional radiology device, said method comprising:
a learning phase consisting in submitting to said multilayer neural network (130) a learning set comprising first input tensors associated with labels, each of said first input tensors comprising data of a first 3D radiology image (31) of a patient in the intervention area and data of first acquisition parameters of the interventional radiology device, said first acquisition parameters (32) comprising three-axis coordinates (x,y,z) of the interventional radiology device, angles, α, β, of orientation of the device, and voltage (KV) of a tube of the interventional radiology device, α representing rotation about a longitudinal axis of the patient, β a rotation about an horizontal axis, each of said labels consisting of a scattered radiation map (33) obtained by simulation by a simulation module (12) from the said first 3D radiology image (31) and said first acquisition parameters (32), said scattered radiation map comprising a 3D image of the operating environment, said 3D image comprising voxels associated with a simulated irradiation dose per unit volume of the operating environment,
iteratively training said multilayer neural network by minimizing a cost function, a predictive model specific to the operating environment being constituted, and
storing said predictive model for use by said trained multilayer neural network for obtaining as output a prediction of a scattered radiation map of said operating environment during interventional radiology of said given patient in said intervention area.

2. The method according to the preceding claim, wherein, during the learning phase, a plurality of data of the learning set is generated (11) for a first 3D radiology image (31) by varying said first acquisition parameters among possible parameters.

3. The method according to any of the preceding claims, wherein said simulation is performed by a Monte-Carlo method adapted for a graphics processor.

4. The method according to any of the preceding claims, wherein said neural network is of U-Net type.

5. The method according to the preceding claim, wherein said neural network (130) consists of a first sub-network (131) having as input the data of said first 3Dradiology image, and comprising a first succession of convolution and pooling layers and a second succession of deconvolution and convolution layers, such that the output of said neural network (130) is of the same size as said input, and a second sub-network (132) having as input said first acquisition parameters, respectively, and whose output is concatenated with the output of the last pooling layer of said first succession.

6. The method according to any of the preceding claims, wherein said first input tensor is a concatenation of said data of a first 3D radiology image (31) and said first acquisition parameters (32).

7. The method according to any of the preceding claims, wherein said radiology image (31) is a computerized tomography scan.

8. Method for generating a predicted scattered radiation map (34) of an operating environment, comprising a step of training a multilayer neural network (130) according to any of the previous claims, and a step of predicting said predicted scattered radiation map (34) during interventional radiology of said intervention area of a given patient using said interventional radiology device, by submitting to said trained multilayer neural network an input tensor comprising data of a 3D radiology image of said given patient in said intervention area, and data of acquisition parameters acquired during the interventional radiology from the interventional radiology device (21), said 3D radiology image and said acquisition parameters being of a same type as those used in the step of training, said prediction of a scattered radiation map being of a same type as the labels of the learning set, and retrieving as output said predicted scattered radiation map (34).

9. Computer readable medium encoding a machine-executable program of instructions to perform a method according to any one of claims 1 to 8.

10. System for generating a predicted scattered radiation map of an operating environment during interventional radiology of an intervention area of a patient using an interventional radiology device, said system comprising a multilayer neural network (130) and being adapted to:
- obtain a stream of acquisition parameters (32) from an interventional radiology device (21), said acquisition parameters comprising three-axis coordinates (x,y,z) of the interventional radiology device, angles, α, β, of orientation of the device, and voltage (KV) of a tube of the interventional radiology device, α representing rotation about a longitudinal axis of the patient, β a rotation about an horizontal axis,
- obtain a 3D radiology image (31) of the patient (130) in the intervention area,
- prepare an input tensor comprising data of the 3D radiology image and of the acquisition parameters,
- submit said input tensor as input to the multilayer neural network (13), said neural network having been trained during a learning phase with a learning set of first input tensors associated with labels, each of said first input tensors being of a same type as said input tensor prepared for the patient and comprising data of a first 3D radiology image (31) of a patient in the intervention area, and first acquisition parameters (32) of the interventional radiology device, each of said labels corresponding to a scattered radiation map (33) of the operating environment obtained from said simulation module (12) on the basis of said first 3D radiology image (31) and said first acquisition parameters (32); and comprising a 3D image of the operating environment comprising voxels associated with a simulated irradiation dose per unit volume of the operating environment, and
- retrieve as an output of the multilayer neural network (13) the predicted scattered radiation map of the operating environment, said scattered radiation map being of a same type as the labels of the learning set.

## Patentansprüche

1. Verfahren zum Trainieren eines mehrschichtigen neuronalen Netzwerks (130) im Hinblick auf das Erhalten einer prädizierten Streustrahlungskarte einer Operationsumgebung während einer interventionellen Radiologie eines Interventionsbereichs eines bestimmten Patienten unter Verwendung einer interventionellen Radiologievorrichtung, wobei das Verfahren umfasst:
eine Lernphase, die darin besteht, dem mehrschichtigen neuronalen Netzwerk (130) einen Lernsatz zuzuführen, der erste Eingabetensoren umfasst, die mit Labels verknüpft sind, wobei jeder der ersten Eingabetensoren Daten eines ersten 3D-Radiologiebildes (31) eines Patienten im Interventionsbereich und Daten von ersten Erfassungsparametern der interventionellen Radiologievorrichtung umfasst, wobei die ersten Erfassungsparameter (32) Drei-Achsen-Koordinaten (x,y,z) der interventionellen Radiologievorrichtung, Ausrichtungswinkel, α, β, der Vorrichtung und eine Spannung (KV) einer Röhre der interventionellen Radiologievorrichtung umfassen, wobei α eine Drehung um eine Längsachse des Patienten darstellt, β eine Drehung um eine horizontale Achse, wobei jedes der Labels aus einer Streustrahlungskarte (33) zusammengesetzt ist, die durch Simulation durch ein Simulationsmodul (12) aus dem ersten 3D-Radiologiebild (31) erhalten wird und den ersten Erfassungsparametern (32), wobei die Streustrahlungskarte ein 3D-Bild der Operationsumgebung umfasst, wobei das 3D-Bild Voxel umfasst, die einer simulierten Bestrahlungsdosis pro Volumeneinheit der Operationsumgebung zugeordnet sind,
iteratives Trainieren des mehrschichtigen neuronalen Netzwerks durch Minimieren einer Kostenfunktion, wobei ein für die Operationsumgebung spezifisches prädiktives Modell gebildet wird, und Speichern des prädiktiven Modells zur Verwendung durch das trainierte mehrschichtige neuronale Netzwerk zum Erhalten, als Ausgabe, einer Prädiktion einer Streustrahlungskarte der Operationsumgebung während einer interventionellen Radiologie des bestimmten Patienten in dem Interventionsbereich.

2. Verfahren nach dem vorstehenden Anspruch, wobei, während der Lernphase, eine Vielzahl von Daten des Lernsatzes für ein erstes 3D-Radiologiebild (31) durch Variieren der ersten Erfassungsparameter unter möglichen Parametern erzeugt (11) wird.

3. Verfahren nach einem der vorstehenden Ansprüche, wobei die Simulation durch ein Monte-Carlo-Verfahren durchgeführt wird, das für einen Grafikprozessor angepasst ist.

4. Verfahren nach einem der vorstehenden Ansprüche, wobei das neuronale Netzwerk von einer U-Net-Art ist.

5. Verfahren nach dem vorstehenden Anspruch, wobei das neuronale Netzwerk (130) aus einem ersten Teilnetz (131), das als Eingabe die Daten des ersten 3D-Radiologiebildes aufweist und eine erste Abfolge von Faltungs- und Pooling-Schichten und eine zweite Abfolge von Entfaltungs- und Faltungsschichten umfasst, sodass die Ausgabe des neuronalen Netzwerks (130) von der gleichen Größe wie die Eingabe ist, und einem zweiten Teilnetz (132) besteht, das jeweils die ersten Erfassungsparameter als Eingabe aufweist und dessen Ausgabe mit der Ausgabe der letzten Pooling-Schicht der ersten Abfolge verkettet ist.

6. Verfahren nach einem der vorstehenden Ansprüche, wobei der erste Eingabetensor eine Verkettung der Daten eines ersten 3D-Radiologiebilds (31) mit den ersten Erfassungsparametern (32) ist.

7. Verfahren nach einem der vorstehenden Ansprüche, wobei das Radiologiebild (31) ein computergestützter Tomografiescan ist.

8. Verfahren zum Erzeugen einer prädizierten Streustrahlungskarte (34) einer Operationsumgebung, umfassend einen Schritt des Trainierens eines mehrschichtigen neuronalen Netzwerks (130) nach einem der vorstehenden Ansprüche, und einen Schritt des Prädizierens der prädizierten Streustrahlungskarte (34) während einer interventionellen Radiologie des Interventionsbereichs eines bestimmten Patienten unter Verwendung der interventionellen Radiologievorrichtung, durch Übermitteln eines Eingabetensors an das trainierte mehrschichtige neuronale Netzwerk, der Daten eines 3D-Radiologiebildes des bestimmten Patienten in dem Interventionsbereich und Daten von Erfassungsparametern, die während der interventionellen Radiologie von der interventionellen Radiologievorrichtung (21) erfasst wurden, umfasst, wobei das 3D-Radiologiebild und die Erfassungsparameter von der gleichen Art sind wie diejenigen, die in dem Schritt des Trainierens verwendet wurden, wobei die Prädiktion einer Streustrahlungskarte von einem gleichen Typ wie die Labels des Lernsatzes sind, und Abrufen der prädizierten Streustrahlungskarte (34) als Ausgabe.

9. Computerlesbares Medium, das ein maschinenausführbares Programm von Anweisungen codiert, um ein Verfahren nach einem der Ansprüche 1 bis 8 durchzuführen.

10. System zum Erzeugen einer prädizierten Streustrahlungskarte einer Operationsumgebung während einer interventionellen Radiologie eines Interventionsbereichs eines Patienten unter Verwendung einer interventionellen Radiologievorrichtung, wobei das System ein mehrschichtiges neuronales Netzwerk (130) umfasst und eingerichtet ist zum:
- Erhalten eines Stroms von Erfassungsparametern (32) aus einer Vorrichtung (21) für interventionelle Radiologie, die Erfassungsparameter umfassend Drei-Achsen-Koordinaten (x,y,z) der Vorrichtung für interventionelle Radiologie, Winkel, α, β, der Ausrichtung der Vorrichtung und Spannung (KV) eines Schlauchs der Vorrichtung für interventionelle Radiologie, wobei α die Drehung um eine Längsachse des Patienten, β eine Drehung um eine horizontale Achse darstellt,
- Erhalten eines 3D-Radiologiebilds (31) des Patienten (130) in dem Interventionsbereich,
- Vorbereiten eines Eingabetensors, umfassend Daten des 3D-Radiologiebilds und der Erfassungsparameter,
- Übergeben des Eingabetensors als Eingabe an das mehrschichtige neuronale Netzwerk (13), wobei das neuronale Netzwerk während einer Lernphase mit einem Lernsatz von ersten Eingabetensoren trainiert worden ist, die mit Labels verknüpft sind, wobei jeder der ersten Eingabetensoren von einem gleichen Typ ist wie der für den Patienten aufbereitete Eingabetensor und Daten eines ersten 3D-Radiologiebildes (31) eines Patienten im Interventionsbereich sowie erste Erfassungsparameter (32) der interventionellen Radiologievorrichtung umfasst, wobei jedes der Labels einer Streustrahlungskarte (33) der Operationsumgebung entspricht, die von dem Simulationsmodul (12) auf der Basis des ersten 3D-Radiologiebildes (31) und der ersten Erfassungsparameter (32) erhalten wurde; und umfassend ein 3D-Bild der Operationsumgebung, umfassend Voxel, die einer simulierten Bestrahlungsdosis pro Volumeneinheit der Operationsumgebung zugeordnet sind, und
- Abrufen als Ausgabe des mehrschichtigen neuronalen Netzes (13) der prädizierten Streustrahlungskarte der Operationsumgebung, wobei diese Streustrahlungskarte vom gleichen Typ ist wie die Labels des Lernsatzes.

## Revendications

1. Procédé permettant d'entraîner un réseau neuronal multicouche (130) en vue de l'obtention d'une carte de rayonnement diffusé prédite d'un environnement opératoire pendant une radiologie interventionnelle d'une zone d'intervention d'un patient donné à l'aide d'un dispositif de radiologie interventionnelle, ledit procédé comprenant :
une phase d'apprentissage consistant à soumettre audit réseau neuronal multicouche (130) un ensemble d'apprentissage comprenant des premiers tenseurs d'entrée associés à des étiquettes, chacun desdits premiers tenseurs d'entrée comprenant des données d'une première image radiologique 3D (31) d'un patient dans la zone d'intervention et des données de premiers paramètres d'acquisition du dispositif de radiologie interventionnelle, lesdits premiers paramètres d'acquisition (32) comprenant des coordonnées à trois axes (x, y, z) du dispositif de radiologie interventionnelle, des angles, α, β, d'orientation du dispositif, et la tension (KV) d'un tube du dispositif de radiologie interventionnelle, α représentant une rotation autour d'un axe longitudinal du patient, β une rotation autour d'un axe horizontal, chacune desdites étiquettes consistant en une carte de rayonnement diffusé (33) obtenue par simulation par un module de simulation (12) à partir de ladite première image radiologique 3D (31) et desdits premiers paramètres d'acquisition (32), ladite carte de rayonnement diffusé comprenant une image 3D de l'environnement opératoire, ladite image 3D comprenant des voxels associés à une dose d'irradiation simulée par unité de volume de l'environnement opératoire,
l'entraînement par itération dudit réseau neuronal multicouche en minimisant une fonction de coût, un modèle prédictif spécifique à l'environnement opératoire étant constitué, et le stockage dudit modèle prédictif pour utilisation par ledit réseau neuronal multicouche entraîné pour l'obtention en guise de sortie d'une prédiction d'une carte de rayonnement diffusé dudit environnement opératoire pendant une radiologie interventionnelle dudit patient donné dans ladite zone d'intervention.

2. Procédé selon la revendication précédente, dans lequel, pendant la phase d'apprentissage, une pluralité de données de l'ensemble d'apprentissage est générée (11) pour une première image radiologique 3D (31) en faisant varier lesdits premiers paramètres d'acquisition parmi des paramètres possibles.

3. Procédé selon l'une quelconque des revendications précédentes, dans lequel ladite simulation est mise en œuvre par un procédé de Monte-Carlo conçu pour un processeur graphique.

4. Procédé selon l'une quelconque des revendications précédentes, dans lequel ledit réseau neuronal est de type U-Net.

5. Procédé selon la revendication précédente, dans lequel ledit réseau neuronal (130) est constitué d'un premier sous-réseau (131) ayant en guise d'entrée les données de ladite première image radiologique 3D, et comprenant une première succession de couches de convolution et de pooling et une seconde succession de couches de déconvolution et de convolution, de telle sorte que la sortie dudit réseau neuronal (130) est de la même taille que ladite entrée, et un second sous-réseau (132) ayant en guise d'entrée lesdits premiers paramètres d'acquisition, respectivement, et dont la sortie est concaténée à la sortie de la dernière couche de pooling de ladite première succession.

6. Procédé selon l'une quelconque des revendications précédentes, dans lequel ledit premier tenseur d'entrée est une concaténation desdites données d'une première image radiologique 3D (31) et desdits premiers paramètres d'acquisition (32).

7. Procédé selon l'une quelconque des revendications précédentes, dans lequel ladite image radiologique (31) est une image de tomodensitométrie.

8. Procédé permettant de générer une carte de rayonnement diffusé prédite (34) d'un environnement opératoire, comprenant une étape d'entraînement d'un réseau neuronal multicouche (130) selon l'une quelconque des revendications précédentes, et une étape de prédiction de ladite carte de rayonnement diffusé prédite (34) pendant une radiologie interventionnelle de ladite zone d'intervention d'un patient donné à l'aide dudit dispositif de radiologie interventionnelle, en soumettant audit réseau neuronal multicouche entraîné un tenseur d'entrée comprenant des données d'une image radiologique 3D dudit patient donné dans ladite zone d'intervention, et des données de paramètres d'acquisition acquis pendant la radiologie interventionnelle auprès du dispositif de radiologie interventionnelle (21), ladite image radiologique 3D et lesdits paramètres d'acquisition étant d'un même type que ceux utilisés dans l'étape d'entraînement, ladite prédiction d'une carte de rayonnement diffusé d'un même type que les étiquettes de l'ensemble d'apprentissage, et la récupération en guise de sortie de ladite carte de rayonnement diffusé prédite (34).

9. Support lisible par ordinateur encodant un programme exécutable par machine d'instructions pour mettre en œuvre un procédé selon l'une quelconque des revendications 1 à 8.

10. Système permettant de générer une carte de rayonnement diffusé prédite d'un environnement opératoire pendant une radiologie interventionnelle d'une zone d'intervention d'un patient à l'aide d'un dispositif de radiologie interventionnelle, ledit système comprenant un réseau neuronal multicouche (130) et étant conçu pour :
- obtenir un flux de paramètres d'acquisition (32) auprès d'un dispositif de radiologie interventionnelle (21), lesdits paramètres d'acquisition comprenant des coordonnées à trois axes (x, y, z) du dispositif de radiologie interventionnelle, des angles, α, β, d'orientation du dispositif, et une tension (KV) d'un tube du dispositif de radiologie interventionnelle, α représentant une rotation autour d'un axe longitudinal du patient, β une rotation autour d'un axe horizontal,
- obtenir une image radiologique 3D (31) du patient (130) dans la zone d'intervention,
- préparer un tenseur d'entrée comprenant des données de l'image radiologique 3D et des paramètres d'acquisition,
- soumettre ledit tenseur d'entrée en guise d'entrée au réseau neuronal multicouche (13), ledit réseau neuronal ayant été entraîné pendant une phase d'apprentissage avec un ensemble d'apprentissage de premiers tenseurs d'entrée associés à des étiquettes, chacun desdits premiers tenseurs d'entrée étant d'un même type que ledit tenseur d'entrée étant préparé pour le patient et comprenant des données d'une première image radiologique 3D (31) d'un patient dans la zone d'intervention, et des premiers paramètres d'acquisition (32) du dispositif de radiologie interventionnelle, chacune desdites étiquettes correspondant à une carte de rayonnement diffusé (33) de l'environnement opératoire obtenue auprès dudit module de simulation (12) en fonction de ladite première image radiologique 3D (31) et desdits premiers paramètres d'acquisition (32) ; et comprenant une image 3D de l'environnement opératoire comprenant des voxels associés à une dose d'irradiation simulée par unité de volume de l'environnement opératoire, et
- récupérer en guise de sortie du réseau neuronal multicouche (13) la carte de rayonnement diffusé prédite de l'environnement opératoire, ladite carte de rayonnement diffusé étant d'un même type que les étiquettes de l'ensemble d'apprentissage.
